# EUROPEAN PATENT SPECIFICATION

(11) **EP 1 650 567 B1**
(45) Date of publication and mention of the grant of the patent: **07.10.2009**
(21) Application number: 04747685.8
(22) Date of filing: 16.07.2004
(51) Int. Cl.: G01N 33/68

(54) **METHOD OF DETECTING PROTEIN DIRT ON CONTACT LENS**
VERFAHREN ZUM NACHWEIS VON PROTEINSCHMUTZ AUF EINER KONTAKTLINSE
PROCEDE POUR DETECTER UNE IMPURETE PROTEIQUE SUR UNE LENTILLE DE CONTACT

(30) Priority: 31.07.2003 JP 2003283356
(43) Date of publication of application: 26.04.2006
(73) Proprietor: Menicon Co., Ltd., Nagoya-shi, Aichi 460-0006 (JP)
(72) Inventor: OHNO, Sadanori; c/o MENICON CO., LTD. Central, Kasugai-shi Aichi; 4870032 (JP); KAWAI, Tetsuji; c/o MENICON CO., LTD. Central, Kasugai-shi Aichi; 4870032 (JP); IWATA, Miyoko; c/o MENICON CO., LTD. Central, Kasugai-shi Aichi; 4870032 (JP); NAKADA, Kazuhiko; c/o MENICON CO., LTD. Central, Kasugai-shi Aichi; 4870032 (JP)
(74) Representative: Paget, Hugh Charles Edward
(86) International application number: PCT/JP2004/010220
(87) International publication number: WO 2005/012915

(56) References cited:
- JP-A- 11 304 803
- JP-A- 11 304 803
- JP-A- 58 222 155
- JP-A- 2000 065 840
- JP-A- 2004 212 813
- US-A- 3 063 812
- GOLDENBERG M S ET AL: "Detection of protein deposition on contact lens type polymeric hydrogels by Coomassie blue R staining." BIOMATERIALS APR 1991, vol. 12, no. 3, April 1991 (1991-04), pages 267-274, XP002443759 ISSN: 0142-9612

## Description

### TECHNICAL FIELD

The present invention relates to a method of detecting protein deposit on a contact lens, in particular, a method of detecting the protein deposit adhering to the contact lens easily and in a short period of time.

### BACKGROUND ART

In recent years, in order to provide intended characteristics, there have been provided contact lenses made of various materials. However, a protein contained in lacrimal fluid tends to adhere to a contact lens, in particular, to a contact lens made of an ionic polymer material obtained by using, as a raw material, monomeric components having ionizable groups. If the protein gradually adheres to and accumulates on surfaces of and inside the contact lens, removal of the protein tends to be difficult. In addition to this, there is an anxiety that the adhesion and the accumulation of the protein may cause various troubles such as deteriorating the wearing comfort as felt by the lens wearer, lowering the oxygen permeability of the lens, lowering the eyesight of the lens wearer, allergy, and corneal injury.

As the methods of quantitatively detecting and determining the protein component adhering to the contact lens, there are conventionally known amino acid analysis, thin layer chromatography, high-performance liquid chromatography, and ELISA method. However, each of these methods requires, before the detection, a process of extraction, in which the deposit adhering to the contact lens is extracted to a predetermined solvent. Accordingly, these methods are not suitable for usual analyses, because the detections are troublesome, taking a long time, or requiring a large and expensive device.

Besides, JP-A-58-222154 (Patent Document 1) and JP-A-58-222155 (Patent Document 2) propose methods of detecting particular components adhering to the contact lenses, by immersing the contact lenses in the solutions for detecting deposits, wherein the solutions include, as a coloring reagent, Nile Blue sulfate or erythrosin. However, these is an anxiety that these coloring reagents may also color lipid component, as well as the protein component, so that there is a problem that the protein component cannot be selectively detected. In addition, JP-A-2000-65840 (Patent Document 3) proposes a method of detecting the protein deposit and the lipid deposit adhering to the contact lens, by using a basic dye solution.

Meanwhile, JP-U-A-5-40874 (Patent Document 4) proposes a reference color spectrum. The Patent Document 4 explains that, there is commonly used tetrabromophenol blue as a color former of a urinary protein test paper, which is used for inspecting the protein components in the urine by means of a method of protein error of a pH indicator. JP-A-8-505938 (Patent Document 5) proposes a test panel which represents an immediate result, wherein bromochlorophenol blue and tetrabromophenol blue are used as visual indicators. The Patent Document 5 explains that an existence or a loss of a substance to be analyzed, e.g., the protein, included in a body fluid such as lacrimal fluid, can be recognized according to a color tone of the indicator. Moreover, JP-A-11-304803 (Patent Document 6) proposes a method and a kit for analyzing the protein in a sample. JP-A-11-304803 discloses a method of analyzing the protein by using an association of triphenylmethane dye color, such as bromochlorophenol blue or tetrabromophenol blue, with the protein, and causing the association to adsorb on a hydrophobic filter. However, these methods disclosed in the Patent Documents 4 to 6 are mainly intended to detect the protein component included in a fluid such as the urine. Therefore, there is no disclosure of a concrete method of detecting the protein component included in the lacrimal fluid adhering to the contact lens.

Patent Document 1: JP-A-58-222154
Patent Document 2: JP-A-58-222155
Patent Document 3: JP-A-2000-65840
Patent Document 4: JP-U-A-5-40874
Patent Document 5: JP-A-8-505938
Patent Document 6: JP-A-11-304803
Goldenberg and Beekman (Biomaterials, Vol 12, page 267-274, 1991) describe a method of detection of protein deposition on contact lens type polymeric hydrogels using Coomassie blue R. Coomassie blue R staining was performed in a solution of water/methanoUacetic acid. Destaining was performed in a solution of water/methanol/acetic acid.

### DISCLOSURE OF THE INVENTION

### PROBLEM TO BE SOLVED BY THE INVENTION

The present invention was made in view of the background art described above. A problem to be solved by the invention is to provide a method of detecting only the protein deposit adhering to a contact lens easily and in a short period of time.

### MEANS FOR SOLVING THE PROBLEM

The present invention was made to solve the problem described above. In first preferred form of the present invention, there is provided a method of detecting protein deposit on a contact lens characterized in that a protein component adhering to the contact lens, after the contact lens has been worn, is detected when the protein component adhering to the contact lens is in its colored state, wherein the protein component is colored by causing the contact lens to contact a solution having pH of not more than 3, after causing the contact lens to contact a coloring reagent solution containing bromochlorophenol blue or tetrabromophenol blue.

Besides, in second preferred form of the present invention, the coloring reagent in the coloring reagent solution has a concentration of 100 - 500 ppm.

Moreover, in third preferred form of the present invention, it is desirable that the coloring reagent solution is obtained by dissolving the coloring reagent in water, ethanol, or a mixture thereof.

In addition, in fourth preferred form of the present invention, it is desirable that the solution having pH of not more than 3 is an aqueous solution of phosphoric acid.

Also, in fifth preferred form of the present invention, as the solution having pH of not more than 3, there is preferably employed a solution, whose pH is within a range of 2 - 3.

Moreover, in sixth preferred form of the method of detecting the protein deposit on the contact lens in accordance with the present invention, the detection of the protein deposit adhering to the contact lens is implemented by a colorimetric analysis, a spectral analysis, or an analysis obtained by combining these analyses.

Furthermore, in seventh preferred form of the present invention, the detection of the protein deposit adhering to the contact lens is implemented by image processing of an image taken with a camera, which image is of the contact lens caused to contact the coloring reagent solution and the solution having pH of not more than 3.

### ADVANTAGEOUS EFFECT OF THE INVENTION

According to the above-mentioned first preferred form of the present invention, bromochlorophenol blue or tetrabromophenol blue is used as the coloring reagent. Owing to this, selective detection of the protein component adhering to the contact lens is advantageously realized, without causing a problem such that a result of the detection is influenced by a kind of the protein component. Namely, the coloring reagent develops a color by associating or reacting with various protein components, without coloring the lipid component.

Besides, in the present invention, the contact lens, after it has been worn, is caused to contact a solution including the coloring reagent (coloring reagent solution), and is further caused to contact a solution whose pH is adjusted to be not more than 3 (acidic solution) by, for example, immersing the contact lens in the solution. Owing to this, color of only the protein component is advantageously developed or given in a short period of time. Accordingly, positions on the contact lens, to which the protein component is adhered to, are colored in green. Accordingly, the protein component can be detected, based on the extent of the development of the color (depth of the color).

As described above, in the present invention, only the protein component adhering to the contact lens can be detected easily, economically, and in a short period of time, by causing the contact lens to contact the coloring reagent solution and the acid solution in due order, without employing conventionally employed methods of detecting the protein, such as amino aid analysis, thin layer chromatography, high-performance liquid chromatography, and ELISA method, which require a long period of time for the detection, troublesome processes for the detection, or a high cost.

Among various contact lenses, particularly large amount of the protein component is adhered to the ionic soft contact lens, and this results in increasing possibility of causing various troubles, such as deteriorating the wearing comfort as felt by the lens wearer, lowering the oxygen permeability of the lens, shortening the lifetime of the lens, lowering the eyesight of the lens wearer, allergy, and corneal injury. Owing to the above described preferred forms of the present invention, the protein deposit adhering to the contact lens, without imposing an economical burden and troublesome tests on the lens user, for thereby effectively preventing the occurrences of the above-mentioned troubles.

Besides, in the second preferred form of the present invention, the concentration of the coloring reagent is within a predetermined range, so that the development of the color of the protein component adhering to the contact lens is further advantageously realized.

Moreover, in the sixth preferred form of the present invention, the detection of the protein deposit adhering to the contact lens is implemented by the colorimetric analysis, the spectral analysis, or the analysis obtained by combining these analyses, and owing to this, the detection of the protein component can be executed more inexpensively, compared with the conventional methods.

In addition to the above, if the detection of the protein deposit adhering to the contact lens is implemented by an image processing of the image taken with the camera, which image is of the contact lens caused to contact the coloring reagent solution and the solution having pH of not more than 3, in accordance with the seventh preferred form of the present invention, the amount of the protein adhering to overall the contact lens can be easily obtained as a numerical value.

### BRIEF DESCRIPTION OF THE DRAWING

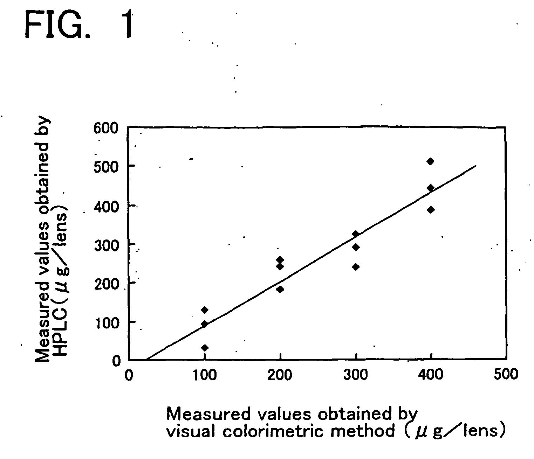

[Fig. 1] This is a graph representing an interrelationship between the results of the quantitative measurement by the colorimetric analysis and those by the size exclusion chromatography, which results are obtained in the EXAMPLE 2.

### BEST MODE FOR CARRYING OUT THE INVENTION

Meanwhile, for the coloring reagent solution to be employed in the present invention, bromochlorophenol blue (BCPB) or tetrabromophenol blue (TBPB) is used as the coloring reagent. Each of these bromochlorophenol blue and tetrabromophenol blue is one of the triphenylmethane dye colors, which turn green by associating or reacting with every kind of the protein. In particular, tetrabromophenol blue has a characteristic of being easy to give color reaction, at a regular rate, with various proteins under room temperature in a short period of time. In other words, tetrabromophenol blue has superiority over other coloring reagents in terms of the determination of the amount of the protein component, so that the tetrabromophenol blue can be preferably adopted. Each of bromochlorophenol blue and tetrabromophenol blue turns yellow if the pH is not more than 3. If the pH exceeds 3, hydrogen of the hydroxyl group dissociates, and bromochlorophenol blue and tetrabromophenol blue turn the same color as that of the association of the protein and the coloring reagent (protein-coloring reagent association product), i.e., bromochlorophenol blue and tetrabromophenol blue turn green.

In the present invention, the coloring reagent solution is prepared by dissolving and including the above-mentioned coloring reagent in a predetermined solvent. If the concentration of the coloring reagent in the coloring reagent solution is too high, the dissolution of the coloring reagent in the solvent becomes difficult. On the other hand, if the concentration of the coloring reagent in the coloring reagent solution is too low, there is an inconvenience, caused by difficulty in developing the color, or by taking a long period of time for the measurement. Therefore, in general, there is employed a concentration within a range of about 1 to about 1000 ppm. In this range of the concentration, it is particularly desirable for the present invention to employ a concentration within a range of 100 to 500 ppm. This is because, if there is arranged the concentration of the coloring reagent to be within the range of 100 to 500 ppm and is used a suitable amount of the coloring reagent solution, the coloring reagent associates with the protein in a short period of time.

The solvent for dissolving the coloring reagent is not particularly limited, as long as it is able to dissolve the coloring reagent. Examples of the solvent include water, methanol, ethanol, acetone, and a mixture of two or more of these solvents. Among these solvents, water, ethanol, and a mixture of water and ethanol are especially preferably used, because these solvents are also used at a hospital, etc..

In addition, various known additives may be suitably selected and added to the coloring reagent according to the present invention. Examples of the additives include: stabilizing agents such as butylhydroxytoluene; and solubilizers such as sodium lauryl sulphate. These additives are used within an amount which does not obstruct the object of the present invention.

The coloring reagent solution can be easily prepared in a way as usually used for preparing a solution, without requiring any special procedure. Namely, the coloring reagent is prepared by dissolving the coloring reagent and additives, which are added as needed, in the solvent.

In the present invention, the protein deposit is detected by using the coloring reagent as obtained above, and detecting or determining the amount of the protein component adhering to the contact lens, in which the protein component is adhered to the contact lens by wearing.

In detail, at first a suitable amount of the coloring reagent solution containing bromochlorophenol blue or tetrabromophenol blue is generally accommodated in a container, which has a size to be able to accommodate the contact lens. In this coloring reagent solution, the contact lens, which has been worn, i.e., the contact lens, to which the protein deposit is adhered, is immersed in a way that overall the contact lens is immersed, so that the contact lens is caused to contact the coloring reagent solution. Accordingly, the various protein components adhering to the contact lens are associated or reacted with the coloring reagent in the coloring reagent solution in a short period of time.

In this case, the period of time for the contact (immersion) is not particularly limited, and is suitably determined according to the concentration, etc., of the coloring reagent solution to be used. However, if the period of time for contacting the contact lens with the coloring reagent solution is too short, it is difficult to give the quantitative association or reaction, so that there is an anxiety that the development of the color cannot be sufficiently achieved. On the other hand, if the period of time for contacting (immersing) the contact lens with the coloring reagent solution is too long, the association or the reaction is excessively progressed, so that there is an anxiety that the protein-coloring reagent association product is eluted or dissolved from the contact lens. Therefore, it is desirable that the period of time for the contact is one second to 10 minutes, and more preferably 1 to 5 minutes.

If the contact lens is taken out of the coloring reagent solution, the coloring reagent solution adheres to overall the surfaces of the contact lens, so that overall the contact lens is colored and turned green. However, if it is left as it is, the color of the coloring reagent is overlapped with the color of the protein -coloring reagent association product, so that it is not possible to distinguish and detect only the colored protein component. Therefore, it is needed to remove the obstruction caused by the coloring reagent adhering to the surfaces of the contact lens. For this reason, in the present invention, the contact lens is immersed in the solution (acid solution), whose pH is adjusted to be not more than 3. In this case, overall the contact lens is immersed in the solution, so as to cause the contact lens to contact the acid solution. By treating the contact lens with the acid solution as described above, the coloring reagent adhering to the surfaces of the contact lens is removed, or the color of the coloring reagent adhering to the contact lens is changed from green to yellow in a short period of time, whereby only the green color of the protein - coloring reagent association product remains.

If the pH of the above-mentioned acid solution is more than 3, the color of the coloring reagent adhering to the contact lens cannot be sufficiently changed. For this reason, it is not possible to advantageously develop the color of only a part of the contact lens, to which the protein is adhered, whereby the detection of the protein deposit cannot be accurately executed. If the contact lens, which has been immersed in the coloring reagent solution, is rinsed with, e.g., distilled water or tap water, of which the pH is not adjusted, in place of the above-mentioned acid solution, the coloring reagent adhering to the contact lens cannot be completely removed, so that the color of the coloring reagent is left on overall the contact lens, whereby it is not possible to visually confirm only the protein-coloring reagent association product. In addition, the lower limit of the pH of the acid solution as described above is not particularly limited. However, if the pH is excessively low, there is an anxiety that the protein or the contact lens material may be decomposed, so that it is desirable that the lower limit of the pH is not less than 2.

In addition, the above-mentioned acid solution is easily prepared by suitably selecting conventionally known pH adjuster or pH buffer and dissolving it in an aqueous solution, as required. Examples of the aqueous solution include water and water-based various solvents, such as a solvent obtained by mixing water and ethanol, etc.. Meanwhile, examples of the pH adjuster or the pH buffer include acids such as phosphoric acid, hydrochloric acid, sulfuric acid, and nitric acid. Among these acids, for the safety reason, an aqueous solution of phosphoric acid is preferably used.

The period of time for contacting (immersing) the contact lens with the acid solution is not particularly limited, as long as it is sufficient to change the color of the coloring reagent adhering to the contact lens to yellow. However, it is desirable that the period of time for the contacting is one second to 10 minutes, preferably 1 to 5 minutes. If the period of time for contacting the contact lens with the acid solution is too short, there is an anxiety that the color of the coloring reagent cannot be completely changed to yellow, and it is tended to be difficult to distinguish the green color of only the protein-coloring reagent association product. On the other hand, if the period of time for contacting (immersing) the contact lens with the acid solution is too long, there is an anxiety that the protein adhering to the contact lens is decomposed by the acid solution.

As described above, the coloring reagent, which is associated with the protein adhering to the contact lens, is colored in green, whereby the protein deposit can be easily detected. This detection may be implemented while the contact lens is existed in the acid solution, or the detection may be implemented after the contact lens has been taken out of the acid solution.

There can be easily and inexpensively detected and determined the protein component adhering to the contact lens, by analyzing the color change of the contact lens, in other words, the depth of the color (degree of the development of the color) of the protein-coloring reagent association product of the contact lens, by using various known methods, such as: visual colorimetric analysis; spectral analysis by using a spectrophotometer; a method which comprises an image processing, such as an accumulation of a luminance based on an image taken with a camera, which is equipped with a sensor such as CCD or CMOS; and a method obtained by combining these methods. Among these analyzing methods, the method of obtaining the luminance accumulation value (luminance accumulation method) by image processing of the image taken with the camera is preferably used, because a total amount of the protein adhering to the contact lens can be obtained as a numerical value. Meanwhile, if there is needed a more convenient or a more inexpensive detection, the visual colorimetric analysis is preferably used.

Where the amount of the adhered protein is detected by the visual colorimetric analysis, there are prepared standard color tone samples (control samples), and the amount of the protein component is detected by visually comparing the contact lens as a subject of the detection with the color tone samples. For example, there are obtained the control samples, by artificially attaching the protein component to the contact lenses, in a similar way to obtain the contact lens as the subject of the detection, such that the respective amounts of the protein component adhering to the contact lenses as the control samples are 0 µg/lens, 100 µg/lens, 200 µg/lens, 300 µg/lens, and 400 µ g/lens.

Where the amount of the adhered protein is detected by the spectral analysis, the amount of the protein can be detected by measuring the absorption under a light of 624 nm, which absorption wavelength (maximum absorption wavelength) is peculiar to the protein-coloring reagent association product, by using the spectrophotometer. Alternatively, the amount of the protein can be detected by obtaining the luminance (the degree of brightness) under a predetermined wavelength band, by taking an image of the contact lens by using an image-taking device such as a color CCD camera, and processing the image taken with the camera.

As is described above, if the protein deposit is detected according to the present invention, only the protein component adhering to the contact lens can be advantageously detected easily, inexpensively, and in a short period of time, by the simple processe of causing the contact lens to contact the coloring reagent solution and the acid solution, in due order.

Therefore, the amount of the protein adhering to the contact lens can be measured, without imposing the economical burden and troublesome tests on the contact lens wearer, there can be advantageously prevented or reduced the occurrence of various troubles such as deteriorating the wearing comfort as felt by the lens wearer, lowering the oxygen permeability of the lens, shortening the lifetime of the lens, lowering the eyesight of the lens wearer, allergy, and corneal injury.

While the presently preferred embodiments of this invention have been described in detail, it should be understood that the invention is not limited to the details of the foregoing description, but may be otherwise embodied.

For example, in the foregoing embodiments, the contact lens was caused to contact the coloring reagent solution or the acid solution by being immerged in the solution. However, the method of the contact is not limited to the "immersing", and each of the solutions may be poured or sprayed to the contact lens, for thereby causing the solution to contact the contact lens.

There will be described some examples of the present invention to further clarify the present invention. It is to be understood, however, that the present invention is not limited to the details of the following examples.

### EXAMPLE 1

First of all, there were prepared, as the contact lenses to be used as specimens, two each of the following four kinds of commercially available contact lenses (A to D). The following contact lenses A and B were made of similar materials, but were manufactured by different companies.
A: HEMA (hydroxyethyl methacrylate)-based ionic high water content soft contact lens
B: HEMA-based ionic high water content soft contact lens
C: N-vinyl pyrrolidone-based nonionic high water content soft contact lens
D: HEMA-based nonionic low water content soft contact lens

One of the each kind of the above-mentioned water-contained soft contact lenses was immersed in 0.7% aqueous solution of lysozym for 16 hours, whereby the protein component (lysozym) was artificially adhered to the contact lenses.

The other one of the each kind of the above-mentioned water-contained contact lenses was not immersed in the aqueous solution of lysozym, and was used as the lens for comparison (the contact lens to which the lysozym is not adhered) A to D.

Thereafter, each of total eight contact lenses, consisting of the contact lenses A to D, to which the protein is adhered, and the lenses for comparison A to D, was immersed in 400 ppm TBPB ethanol solution at room temperature for one minute, and was taken out of the solution. Subsequently, each of the contact lenses was immersed in 10 mmol/L aqueous solution of phosphoric acid (the pH of the solution is about 2 to about 3) for not less than 30 seconds. In this way, the color of the protein component adhering to the contact lens was developed.

The colors of the contact lenses were visually observed, while the contact lenses were still immersed in the solution. As a result, there was confirmed that the green color was not developed on each of the lenses for comparison A to D, to which the protein was not adhered. The contact lens A, which was immersed in the aqueous solution of lysozym, exhibited a distinctively deep green color, compared with the lens for comparison A. The contact lens B, which was immersed in the aqueous solution of lysozym, exhibited a slightly green color, compared with the lens for comparison B. The contact lenses C and D, which were immersed in the aqueous solution of lysozym, did not exhibit difference in the color, compared with the lenses for comparison C and D. According to these results, there was confirmed that the protein deposit could be easily detected by the visual colorimetric analysis.

Besides, the eight contact lenses, which were immersed in the TBPB ethanol solution and the aqueous solution of phosphoric acid, in due order, as described above, were imaged with a digital camera, which was equipped with a CCD, while the contact lenses were still immerged in the solution. Thus obtained images taken with the digital camera were imported to a computer. The luminance accumulation value of the each kind of the contact lenses was obtained as the difference between the luminance, under a light of 624nm, of each of the protein adhering lenses and that of the corresponding lens for comparison, by processing the image of the each kind of the contact lenses, by using an image processing soft "AQUACOSMOS" available from KABUSHIKI KAISHA HAMAMATSU PHOTONICS. Thus obtained luminance accumulation values of the contact lenses A, B, C, and D were 5058, 999, 111, and 79, respectively. In this way, the protein deposit adhering to the contact lens can be described in numerical values, and even some kinds of the protein deposits, which were difficult to be detected by the visual colorimetric analysis, could be detected.

### EXAMPLE 2

Two kinds of the above-mentioned commercially available water-contained contact lenses A and C were obtained, and each of these contact lenses were respectively worn by each of the three contact lens wearers a to c for one hour, three hours, five hours, and one day. Thus obtained worn contact lenses were immersed in 400 ppm TBPB ethanol solution at room temperature for one minute, and then the contact lenses were immerged in 10 mmol/L aqueous solution of phosphoric acid for not less than 30 seconds. In this way, the color of the protein deposit adhering to the contact lenses was developed. These lenses were visually observed, and there were found that the lens A turned green and the green color of the lens A became deeper, as the duration of the wearing increases, while the color of the lens C was hardly developed. There were also executed the visual colorimetric analyses with respect of the lens A. The results are shown in TABLE 2. As the standard color tone samples (control samples), there were prepared the control lenses, to which the protein component was artificially attached (0µ g/lens, 100µg/lens, 200µg/lens, 300µg/lens, and 400µg/lens), and the colors of the contact lenses were developed in the same way as the development of the color of the above-described lenses A and C. The colorimetric analysis was executed by comparing the colors of the contact lenses A and C with those of the control samples.

Meanwhile, similar to the above, each of the two kinds of the commercially available contact lenses A and C was respectively worn by each of the above-mentioned three contact lens wearers for one hour, three hours, five hours, and one day. The each of the worn contact lenses was separately accommodated in a vial container, together with 1mL extract (0.2% aqueous solution of trifluoroacetic acid/acetonitrile = 50/50), and the protein adhering to the contact lens was extracted. After the extraction, the extract was analyzed by a size exclusion chromatography ("Alliance 2690" available from Waters Corporation), by using a detector and conditions as described below. In this way, the amounts of the adhering protein were quantitatively measured. The results are shown in the following TABLE 1 and TABLE 2. In the TABLE 1, there are shown the average values of the measured values, while in the TABLE 2, there are only shown the amounts of the protein adhering to the lens A. Moreover, there are plotted the relationships between the quantitatively measured values obtained by the size exclusion chromatography (HPLC) and the above-mentioned measured values obtained by the colorimetric analysis (see Fig. 1).
PDA (Photodiode Array) Detector:
   "PDA 996" available from Waters Corporation in USA
Column: .
   "TSK-GEL G3000SWXL" 7.8 X 300mm available from TOSOH CORPORATION in JAPAN
Temperature of the oven: 30°C
Moving phase:
   0.5 mol/L sodium chloride+0.1 mol/L phosphate buffer (pH 5)
Flow rate: 0.5 mL/minute

[TABLE 1]

| Duration of wearing | One hour | | Three hours | | Five hours | | One day | |
|---|---|---|---|---|---|---|---|---|
| Kind of the lens | A | C | A | C | A | C | A | C |
| Measured values obtained by HPLC* (µg/lens) | 84 | 0.1 | 229 | 0.3 | 286 | 0.2 | 448 | 0.3 |

| | | | | | | | | |
|---|---|---|---|---|---|---|---|---|
| * Each of the values is average value obtained from the three people: a to c. | | | | | | | | |

**[TABLE 2]**

| Duration of wearing | Wearer | Measured values obtained by visual colorimetric analysis (µg/lens) | Measured values obtained by HPLC (µg/lens) |
|---|---|---|---|
| One hour | a | 100 | 30 |
| | b | 100 | 130 |
| | c | 100 | 92 |
| Three hours | a | 200 | 260 |
| | b | 200 | 185 |
| | c | 200 | 243 |
| Five hours | a | 300 | 240 |
| | b | 300 | 291 |
| | c | 300 | 326 |
| One day | a | 400 | 512 |
| | b | 400 | 389 |
| | c | 400 | 444 |

As is apparent from the TABLE 2 and the Fig. 1, there were confirmed excellent interrelationships between the results of the quantitative measurement of the amount of the protein obtained by the coloring reagent (visual colorimetric analysis) and the quantitative measurement of the amount of the protein obtained by the size exclusion chromatography, whereby there is recognized that the measurement method by using the colorimetric reagent is effective. In addition, as is apparent from the above TABLE 1, the relationships between the duration of the wearing and the amount of the adhering protein component are varied, depending on the kinds of the contact lenses. Therefore, there is regarded that the troubles, which are caused by the accumulation of the protein component (allergy, deterioration of the eyesight, and deterioration of the wearing comfort) can be advantageously forecasted.

## Claims

1. A method of detecting protein deposit on a contact lens
**characterized in that**:
a protein component adhering to the contact lens, after said contact lens has been worn, is detected when the protein component adhering to the contact lens is in its colored state, wherein the protein component is colored by causing said contact lens to contact a solution having pH of not more than 3, after causing said contact lens to contact a coloring reagent solution containing bromochlorophenol blue or tetrabromophenol blue.

2. A method of detecting protein deposit on a contact lens according to claim 1, wherein said coloring reagent in the coloring reagent solution has a concentration of 100 - 500 ppm.

3. A method of detecting protein deposit on a contact lens according to claim 1 or 2, wherein said coloring reagent solution is obtained by dissolving the coloring reagent in water, ethanol, or a mixture thereof.

4. A method of detecting protein deposit on a contact lens according to any one of claims 1 to 3, wherein said solution having pH of not more than 3 is an aqueous solution of phosphoric acid.

5. A method of detecting protein deposit on a contact lens according to any one of claims 1 to 4, wherein pH of said solution having pH of not more than 3 is within a range of 2 - 3.

6. A method of detecting protein deposit on a contact lens according to any one of claims 1 to 5, wherein said detection of the protein deposit adhering to the contact lens comprises a colorimetric analysis, a spectral analysis, or an analysis obtained by combining said analyses.

7. A method of detecting protein deposit on a contact lens according to any one of claims 1 to 5, wherein said detection of the protein deposit adhering to the contact lens comprises image processing of an image taken with a camera, which image is of the contact lens caused to contact the coloring reagent solution and the solution having pH of not more than 3.

## Patentansprüche

1. Verfahren zur Detektion von Proteinablagerungen auf einer Kontaktlinse, **dadurch gekennzeichnet, dass**:
eine an der Kontaktlinse haftende Proteinkomponente nach dem Tragen der Kontaktlinse detektiert wird, wenn die an der Kontaktlinse haftende Proteinkomponente in gefärbtem Zustand vorliegt, worin die Proteinkomponente gefärbt wird, indem die Kontaktlinse mit einer Lösung mit einem pH von nicht mehr als 3 in Kontakt gebracht wird, nachdem die Kontaktlinse mit einer Färbereagenslösung in Kontakt gebracht wurde, die Bromchlorphenolblau oder Tetrabromphenolblau enthält.

2. Verfahren zur Detektion von Proteinablagerungen auf einer Kontaktlinse nach Anspruch 1, worin das Färbereagens in der Färbereagenslösung eine Konzentration von 100 bis 500 ppm aufweist.

3. Verfahren zur Detektion von Proteinablagerungen auf einer Kontaktlinse nach Anspruch 1 oder 2, worin die Färbereagenslösung durch Lösen des Färbereagens in Wasser, Ethanol oder einem Gemisch davon erhalten wird.

4. Verfahren zur Detektion von Proteinablagerungen auf einer Kontaktlinse nach einem der Ansprüche 1 bis 3, worin die Lösung mit einem pH von nicht mehr als 3 eine wässrige Phosphorsäurelösung ist.

5. Verfahren zur Detektion von Proteinablagerungen auf einer Kontaktlinse nach einem der Ansprüche 1 bis 4, worin der pH der Lösung mit einem pH von nicht mehr als 3 im Bereich von 2 bis 3 liegt.

6. Verfahren zur Detektion von Proteinablagerungen auf einer Kontaktlinse nach einem der Ansprüche 1 bis 5, worin die Detektion der an der Kontaktlinse haftenden Proteinablagerung eine kolorimetrische Analyse, eine Spektralanalyse oder eine durch Kombination der zuvor genannten Analysen erhaltene Analyse umfasst.

7. Verfahren zur Detektion von Proteinablagerungen auf einer Kontaktlinse nach einem der Ansprüche 1 bis 5, worin die Detektion der an der Kontaktlinse haftenden Proteinablagerung die Bildverarbeitung eines mit einer Kamera aufgenommenen Bilds umfast, wobei das Bild ein Bild der Kontaktlinse ist, die mit der Färbereagenslösung und der Lösung mit einem pH von nicht mehr als 3 in Kontakt gebracht wurde.

## Revendications

1. Procédé pour détecter un dépôt de protéines sur une lentille de contact, **caractérisé en ce que**:
un composant protéique adhérant à la lentille de contact, après que ladite lentille de contact a été portée, est détecté lorsque le composant protéique adhérant à la lentille de contact se trouve dans son état coloré, où le composant protéique est coloré en amenant ladite lentille de contact à venir en contact avec une solution ayant un pH non supérieur à 3, après amener ladite lentille de contact de venir en contact avec une solution de réactif colorant contenant du bleu de bromochlorophénol ou du bleu de tétrabromophénol.

2. Procédé de détection d'un dépôt de protéine sur une lentille de contact selon la revendication 1, dans lequel ledit réactif colorant dans la solution de réactif colorant a une concentration de 100-500 ppm.

3. Procédé pour détecter un dépôt de protéine sur une lentille de contact selon la revendication 1 ou 2, dans lequel ladite solution de réactif colorant est obtenue en dissolvant le réactif colorant dans de l'eau, éthanol ou un mélange de ceux-ci.

4. Procédé pour détecter un dépôt de protéine sur une lentille de contact selon l'une quelconque des revendications 1 à 3, dans lequel ladite solution ayant un pH non supérieur à 3 est une solution aqueuse d'acide phosphorique.

5. Procédé pour détecter un dépôt de protéine sur une lentille de contact selon l'une quelconque des revendications 1 à 4, dans lequel le pH de ladite solution ayant un pH non supérieur à 3 est dans une plage de 2-3.

6. Procédé pour détecter un dépôt de protéine sur une lentille de contact selon l'une quelconque des revendications 1 à 5, dans lequel ladite détection du dépôt de protéine adhérant à la lentille de contact comprend une analyse colorimétrique, une analyse spectrale ou bien une analyse obtenue en combinant lesdites analyses.

7. Procédé pour détecter un dépôt de protéine sur une lentille de contact selon l'une quelconque des revendications 1 à 5, dans lequel ladite détection du dépôt de protéine adhérant à la lentille de contact comprend le traitement d'une image prise avec une caméra, ladite image est de la lentille de contact amenée à venir en contact avec la solution de réactif colorant, et la solution ayant un pH non supérieur à 3.
